# EUROPEAN PATENT APPLICATION

(11) **EP 3 711 613 A1**
(43) Date of publication of application: **23.09.2020**
(21) Application number: 19164473.1
(22) Date of filing: 21.03.2019
(51) Int. Cl.: A24F 47/00, A61M 11/04, A61M 15/06

(54) **AEROSOL DELIVERY DEVICE**

(71) Applicant: NERUDIA LIMITED, Liverpool Merseyside L24 9HP (GB)
(72) Inventor: The designation of the inventor has not yet been filed
(74) Representative: Mewburn Ellis LLP

(57) **Abstract**

An aerosol delivery device comprises a fluid-transfer article, and at least a first casing. The fluid-transfer article includes a reservoir for holding an aerosol precursor, a wick which receives aerosol precursor from that reservoir, and a wick support element. The reservoir is within the first casing, and the wick support element is integral with that first casing. The wick support element has at least one bore therethrough for the passage of aerosol precursor from the reservoir to the wick. Thus, the wick is supported by a component which may be formed simultaneously with the structure which contains the reservoir, which component also allows aerosol precursor to pass from the reservoir to the wick. The aerosol delivery device also has a heater, which makes abutting unbonded contact with an activation surface of the wick so as to interact thermally with that activation surface. Thus, when the heater is active, aerosol precursor which reaches the activation surface will be heated, from the vapour which then may pass to the user.

## Description

### Field of the Invention

The present invention relates to an aerosol delivery device and to an aerosol-generation apparatus for an aerosol delivery device. The present invention preferably relates to an aerosol delivery device including a heater arranged to heat an aerosol precursor to generate an aerosolised composition for inhalation by a user, and to an aerosol-generation apparatus therefor.

### Background

A smoking-substitute device or system is an electronic device that permits the user to simulate the act of smoking by producing an aerosol mist or vapour that is drawn into the lungs through the mouth and then exhaled. The inhaled aerosol mist or vapour typically bears nicotine and/or other flavourings without the odour and health risks associated with traditional smoking and tobacco products. In use, the user experiences a similar satisfaction and physical sensation to those experienced from a traditional smoking or tobacco product, and exhales an aerosol mist or vapour of similar appearance to the smoke exhaled when using such traditional smoking or tobacco products.

One approach for a smoking substitute device is the so-called "vaping" approach, in which a vaporisable liquid, typically referred to (and referred to herein) as "e-liquid", is heated by a heater to produce an aerosol vapour which is inhaled by a user. The e-liquid typically includes a base liquid as well as nicotine and/or flavourings. The resulting vapour therefore also typically contains nicotine and/or flavourings. The base liquid may include propylene glycol and/or vegetable glycerine.

A typical vaping smoking substitute device includes a mouthpiece, a power source (typically a battery), a tank for containing e-liquid, as well as a heater. In use, electrical energy is supplied from the power source to the heater, which heats the e-liquid to produce an aerosol (or "vapour") which is inhaled by a user through the mouthpiece.

Vaping smoking substitute devices can be configured in a variety of ways. For example, there are "closed system" vaping smoking substitute devices, which typically have a sealed tank and heating element. The tank is pre-filled with e liquid and is not intended to be refilled by an end user. One subset of closed system vaping smoking substitute devices include a main body which includes the power source, wherein the main body is configured to be physically and electrically coupled to a consumable including the tank and the heater. The consumable may also be referred to as a cartomizer. In this way, when the tank of a consumable has been emptied, that consumable is disposed of. The main body can be reused by connecting it to a new, replacement, consumable. Another subset of closed system vaping smoking substitute devices are completely disposable, and intended for one-use only.

There are also "open system" vaping smoking substitute devices which typically have a tank that is configured to be refilled by a user. In this way the device can be used multiple times.

An example vaping smoking substitute device is the myblu™ e-cigarette. The myblu™ e cigarette is a closed system device which includes a main body and a consumable. The main body and consumable are physically and electrically coupled together by pushing the consumable into the main body. The main body includes a rechargeable battery. The consumable includes a mouthpiece, a sealed tank which contains e-liquid (also referred to as an aerosol precursor), as well as a heater, which for this device is a heating filament coiled around a portion of a wick. The wick is partially immersed in the e-liquid, and conveys e-liquid from the tank to the heating filament. The device is activated when a microprocessor on board the main body detects a user inhaling through the mouthpiece. When the device is activated, electrical energy is supplied from the power source to the heater, which heats e-liquid from the tank to produce a vapour which is inhaled by a user through the mouthpiece.

For a smoking substitute device it is desirable to deliver nicotine into the user's lungs, where it can be absorbed into the bloodstream. As explained above, in the so-called "vaping" approach, "e-liquid" is heated by a heating device to produce an aerosol vapour which is inhaled by a user. Many e-cigarettes also deliver flavour to the user, to enhance the experience. Flavour compounds are contained in the e-liquid that is heated. Heating of the flavour compounds may be undesirable as the flavour compounds are inhaled into the user's lungs. Toxicology restrictions are placed on the amount of flavour that can be contained in the e-liquid. This can result in some e-liquid flavours delivering a weak and underwhelming taste sensation to consumers in the pursuit of safety.

In aerosol delivery devices, it is desirable to avoid large liquid droplets reaching a user's mouth.

The present invention has been devised in light of the above considerations.

### Summary of the Invention

At its most general, the present invention proposes that an aerosol delivery device has a fluid-transfer article with a wick, with the wick being supported by a wick support element which is integral with a casing of the aerosol delivery device. The fluid-transfer article will also normally comprise a reservoir holding an aerosol precursor, with that reservoir being within the casing. The aerosol delivery device may also have a heater, which makes contact with an activation surface of the wick so as to interact thermally with the activation surface, the wick preferably making abutted unbonded contact with the activation surface.

In this way, the manufacture of the aerosol delivery device may be simplified because the wick is supported by an element which may be moulded at the same time as the moulding of the casing. Separability of the wick and the wick support element from the heater allows the fluid-transfer article to be replaced, for example when the aerosol precursor therein is consumed, without having to replace the heater.

Thus, the present invention may provide an aerosol delivery device comprising a heater, a fluid-transfer article and a first casing, the fluid-transfer article comprising a reservoir for holding an aerosol precursor, a wick arranged to receive aerosol precursor from said reservoir, and a wick support element; wherein said wick support element is arranged to support said wick such that said heater makes abutting unbonded contact with an activation surface of said wick so as to interact thermally with said activation surface; and wherein said first casing contains said reservoir therein and said wick support element is integral with said first casing, said wick support element having at least one bore therethrough for passage of said aerosol precursor from said reservoir to said wick.

Preferably, the activation surface of the wick is planar.

One possibility within the present invention is for the or each bore through the wick support element to be a non-capillary duct. This allows the aerosol precursor to flow in a non-capillary manner from the reservoir to the wick through the bore or bores.

Another possibility is for the or each bore to be a capillary duct (also referred to herein as a capillary bore), so that the flow of aerosol precursor therethrough is controlled by capillary action. In such a case, the or each bore forming a capillary duct may have a diameter of at least 0.3mm, more preferably 0.5mm, but preferably not greater than 2mm. For example, the or each bore may have a diameter of 0.8 to 1.5mm. If the or each bore is a capillary duct, the flow of aerosol precursor therethrough will be influenced by the length of the or each bore, which length is determined by the thickness of the wick support element. It is preferable in such circumstances that the wick support element has a thickness of at least 0.5mm, more preferably at least 1 mm between the reservoir and the wick. It is also preferable that the wick support element has a thickness not greater than 5mm between the reservoir and the wick. Greater thicknesses may limit the amount of aerosol precursor which reaches the wick.

Preferably, the wick support element forms on the wall of the reservoir.

Preferably, the wick is of silica material. In order to form the casing containing the reservoir, it may comprise first and second casing parts, each being hollow and containing the reservoir. In this case, the wick support element may integral with the second casing part. Preferably, the first and second casing parts are separable.

The aerosol delivery device may further include a second casing supporting the heater, with the first and second casings being separably interconnected. The abutting unbonded contact between the heater and the wick allows the heater to be separated from the wick, and remain with the second casing, when the first casing is removed from the second casing.

In such a structure, it is preferable that the first casing has an outlet, with there being a first air-flow pathway from the activation surface to that outlet. In such an arrangement, it is desirable that the wick and the wick support element have aligned openings therethrough, with the aligned openings forming a part of the first air-flow pathway. This is allows air, and also vapour formed by heating of the aerosol precursor, to pass from the activation surface through the wick and the wick support element to the outlet. The second casing may then have an inlet, with a second air-flow pathway from the inlet to the activation surface. The outlet then forms a mouthpiece for the user, air will be drawn through the inlet and the second air-flow pathway to the activation surface when the user sucks or draws on the mouthpiece, and the air can be mixed with heated aerosol precursor, which then passes through the aligned openings in the wick and the wick support element and along the first air-flow pathway to the outlet forming the mouthpiece.

### Summary of the Figures

So that the invention may be understood, and so that further aspects and features thereof may be appreciated, embodiments illustrating the principles of the invention will now be discussed in further detail with reference to the accompanying figures, in which:
**Figure 1** shows a schematic drawing of a first arrangement of a smoking substitute system;
**Figure 2** shows another schematic drawing of the first arrangement of the smoking substitute system;
**Figure 3** shows a schematic drawing of a second arrangement of a smoking substitute system;
**Figure 4** shows another schematic drawing of the second arrangement of the smoking substitute system;
**Figure 5** shows a cutaway view of part of a third arrangement of a smoking substitute system;
**Figure 6** shows a cross-sectional view of an arrangement of a flavour pod;
**Figure 7** shows in detail parts of another arrangement of a smoking substitute system;
**Figure 8** shows detail of the heater and the heater support in the arrangement of Figure 7;
**Figure 9** shows another arrangement of a smoking substitute system;
**Figure 10** shows detail of part of a smoking substitute system;
**Figure 11** shows detail of a heater support which may be used in a smoking substitute system;
**Figure 12** shows detail of an alternative heater support which may be used in a smoking substitute system;
**Figure 13** shows detail of a heater which may be used in a smoking substitute system;
**Figure 14** shows yet another arrangement of a smoking substitute system;
**Figure 15** shows a detailed schematic sectional view of a part of a smoking substitute system;
**Figure 16** shows yet another arrangement of a smoking substitute system;
**Figure 17** shows a consumable part of another smoking substitute system.
**Figure 18** shows another consumable part of a smoking substitute system; and
**Figure 19** shows detail of the consumable part of Figure 18.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

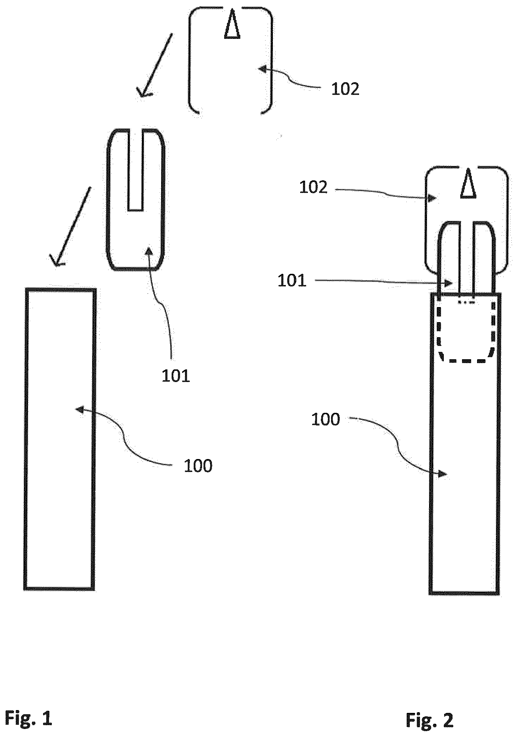

Referring to Figures 1 and 2, there is shown a smoking substitute system comprising a smoking substitute device 100. In this example, the substitute smoking system comprises a cartomiser 101 and a flavour pod 102. The cartomiser 101 may engage with the smoking substitute device 100 via a push-fit engagement, a screw-thread engagement, or a bayonet fit, for example. A cartomiser may also be referred to as a "pod". The smoking substitute system may be an aerosol delivery device according to the present invention.

The flavour pod 102 is configured to engage with the cartomiser 101 and thus with the substitute smoking device 100. The flavour pod 102 may engage with the cartomiser 101 via a push-fit engagement, a screw-thread engagement, or a bayonet fit, for example. Fig. 2 illustrates the cartomiser 101 engaged with the substitute smoking device 100, and the flavour pod 102 engaged with the cartomiser 101. As will be appreciated, in this example, the cartomiser 101 and the flavour pod 102 are distinct elements. Each of the cartomiser 101 and the flavour pod may be an aerosol delivery device.

As will be appreciated from the following description, the cartomiser 101 and the flavour pod 102 may alternatively be combined into a single component that implements the functionality of the cartomiser 101 10 and flavour pod 102. Such a single component may also be an aerosol delivery device according to the present invention. In other examples, the cartomiser may be absent, with only a flavour pod 102 present, or vice versa.

A "consumable" component may mean that the component is intended to be used once until exhausted, and then disposed of as waste or returned to a manufacturer for reprocessing.

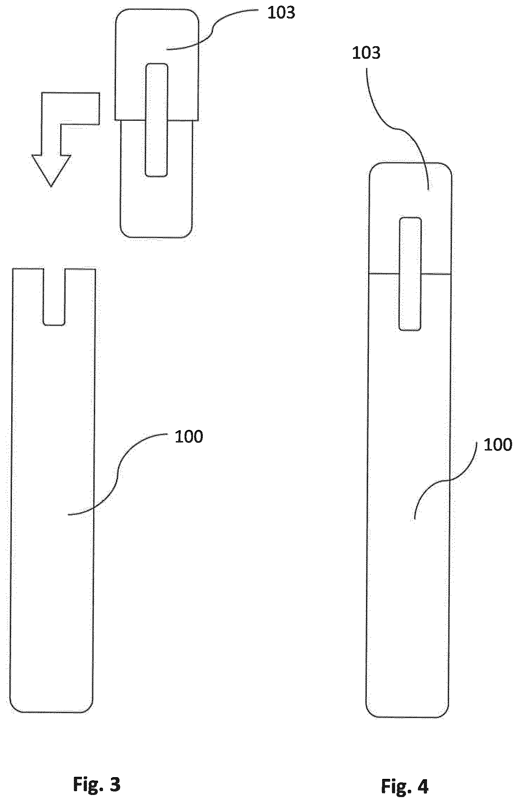

Referring to Figures 3 and 4, there is shown a smoking substitute system comprising a smoking substitute device 100 and a consumable 103. The consumable 103 combines the functionality of the cartomiser 101 and the flavour pod 102. In Figure 3, the consumable 103 and the smoking substitute device 100 are shown separated from one another. In Figure 4, the consumable 103 and the smoking substitute device 100 are engaged with each other.

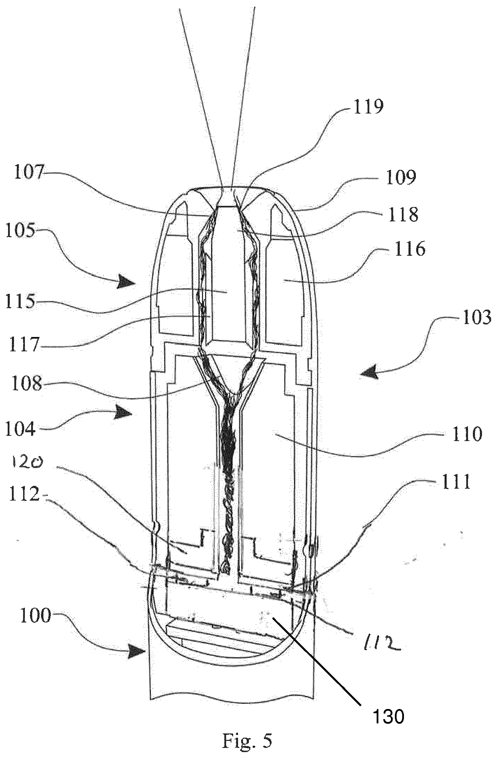

Referring to Figure 5, there is shown a consumable 103 engaged with a smoking substitute device 100 via a push-fit engagement. The consumable 103 may be considered to have two portions - a cartomiser portion 104 and a flavour pod portion 105, both of which are located within a single component (as in Figures 3 and 4).

The consumable 103 includes an upstream airflow inlet 106 and a downstream airflow outlet 107. In other examples a plurality of inlets and/or outlets are included. Between and fluidly connecting the inlet 106 and the outlet 107 there is an airflow passage 108. The outlet 107 is located at the mouthpiece 109 of the consumable 103, and is formed by a mouthpiece aperture.

As above, the consumable 103 includes a flavour pod portion 105. The flavour pod portion 105 is configured to generate a first (flavour) aerosol for output from the outlet 107 of the mouthpiece 109 of the consumable 103. The flavour pod portion 105 of the consumable 103 includes a member 115. The member 115 acts as a passive aerosol generator (i.e. an aerosol generator which does not use heat to form the aerosol, also referred to as a "first aerosol generator" in this example), and is formed of a porous material. The member 115 comprises a supporting portion 117, which is located inside a housing, and an aerosol generator portion 118, which is located in the airflow passage 108. In this example, the aerosol generator portion 118 is a porous nib.

A first storage reservoir 116 (in this example a tank) for storing a first aerosol precursor (i.e. a flavour liquid) is fluidly connected to the member 115. The porous nature of the member 115 means that flavour liquid from the first storage 116 is drawn into the member 115. As the first aerosol precursor in the member 115 is depleted in use, further flavour liquid is drawn from the first storage reservoir 116 into the member 115 via a wicking action.

As described above, the aerosol generator portion 118 is located within the airflow passage 108 through the consumable 103. The aerosol generator portion 118 therefore constricts or narrows the airflow passage 108. The aerosol generator portion 118 occupies some of the area of the airflow passage, resulting in constriction of the airflow passage 108. The airflow passage 108 is narrowest adjacent to the aerosol generator portion 118. Since the constriction results in increased air velocity and corresponding reduction in air pressure at the aerosol generator portion 118, the constriction is a Venturi aperture 119.

The cartomiser portion 104 of the consumable 103 includes a second storage reservoir 110 (in this example a tank) for storing a second aerosol precursor (i.e. e-liquid, which may contain nicotine). At one end of the second storage reservoir 110 is a wick support element 120, which supports a wick 111. As will be described in more detail later, aerosol precursor passes through one or more bores (not shown in Fig.5) in the wick support element 120 to reach the wick 111. The surface of the wick furthest from the reservoir then acts as an activation surface from which aerosol precursor will be released in the form of a vapour, or a mixture of vapour and aerosol.

A heater 112 is a configured to heat the wick 111. The heater 112 may be in the form of one or more resistive heating filaments that abut the wick 111. The wick 111, the heater 112 and the e-liquid storage reservoir 110 together act as an active aerosol generator (i.e. an aerosol generator which uses heat to form the aerosol, referred to as a "second aerosol generator" in this example). The second storage reservoir 110, the wick support element, and the wick 111 form a fluid-transfer article, as they transfer aerosol precursor to the activation surface to be heated by the heater 112.

The heater 112 is supported in the smoking substitute device 100 by a heater support element 130. There may be one or more passages (not shown in Figure 5) through the heater support element 130 to allow air to reach the activation surface of the wick 111 from an inlet (again not shown in Figure 5) of the smoking substitute device.

The smoking substitute device 100 includes an electrical power source (not shown), for example a battery. That battery is then connected via suitable electrical connections to the heater 112. The heater 112, the battery, and other components of the smoking substitute system device 100 form a non-consumable part of the device from which the consumable may be connected and disconnected.

In the arrangement of the smoking substitute device 100 of Figure 5, and in the arrangement to be described later, the consumable 103 is separable from the rest of the smoking substitute device 100. This allows the consumable 103 to be replaced, or possibly refilled, when the first and/or second aerosol precursor have been consumed by the user. Since the consumable 103 includes the wick 111 and the wick support element 120, these components will be removed when the consumable 103 is separated from the rest of the smoking substitute device 100. The heater 112, on the other hand, will remain when the consumable 103 is removed, so that it is non-consumable

In use, a user draws (or "sucks", or "pulls") on the mouthpiece 109 of the consumable 103, which causes a drop in air pressure at the outlet 107, thereby generating air flow through the inlet, through the passages in the heater support element 130, past the activation surface of the wick 111, along the airflow passage 108, out of the outlet 107 and into the user's mouth.

When the heater 112 is activated (by passing an electric current through one or more heating filaments in response to the user drawing on the mouthpiece 109) the e-liquid (aerosol precursor) located in the wick 111 at the activation surface adjacent to the 0r each heating filament is heated and vaporised to form a vapour. The vapour condenses to form the second aerosol within the airflow passage 108. Accordingly, the second aerosol is entrained in an airflow along the airflow flow passage 108 to the outlet 107 and ultimately out from the mouthpiece 109 for inhalation by the user when the user 10 draws on the mouthpiece 109.

The substitute smoking device 100 supplies electrical current to the heating filament or filaments of the heater 112 and the heating filament or filaments heat up. As described, the heating of the heating filament or filaments causes vaporisation of the e-liquid in the wick 111 to form the second aerosol.

As the air flows up through the airflow passage 108, it encounters the aerosol generator portion 118. The constriction of the airflow passage 108 caused by the aerosol generator portion 118 results in an increase in air velocity and corresponding decrease in air pressure in the airflow in the vicinity of the porous surface 118 of the aerosol generator portion 115. The corresponding low pressure region causes the generation of the first (flavour) aerosol from the porous surface 118 of the aerosol generator portion 118. The first (flavour) aerosol is entrained into the airflow and ultimately is output from the outlet 107 of the consumable 103 and thus from the mouthpiece 109 into the user's mouth.

The first aerosol may be sized to inhibit pulmonary penetration. The first aerosol may be formed of particles with a mass median aerodynamic diameter that is greater than or equal to 15 microns, in particular, greater than 30 microns, more particularly greater than 50 microns, yet more particularly greater than 60 microns, and even more particularly greater than 70 microns.

The first aerosol may be sized for transmission within at least one of a mammalian oral cavity and a mammalian nasal cavity. The first aerosol may be formed by particles having a maximum mass median aerodynamic diameter that is less than 300 microns, in particular less than 200 microns, yet more particularly less than 100 microns. Such a range of mass median aerodynamic diameter will produce aerosols which are sufficiently small to be entrained in an airflow caused by a user drawing air through the flavour element and to enter and extend through the oral and or nasal cavity to activate the taste and/or olfactory receptors.

The second aerosol generated may be sized for pulmonary penetration (i.e. to deliver an active ingredient such as nicotine to the user's lungs). The second aerosol may be formed of particles having a mass median aerodynamic diameter of less than or equal to 10 microns, preferably less than 8 microns, more preferably less than 5 microns, yet more preferably less than 1 micron. Such sized aerosols tend to penetrate into a human user's pulmonary system, with smaller aerosols generally penetrating the lungs more easily. The second aerosol may also be referred to as a vapour.

The size of aerosol formed without heating is typically smaller than that formed by condensation of a vapour.

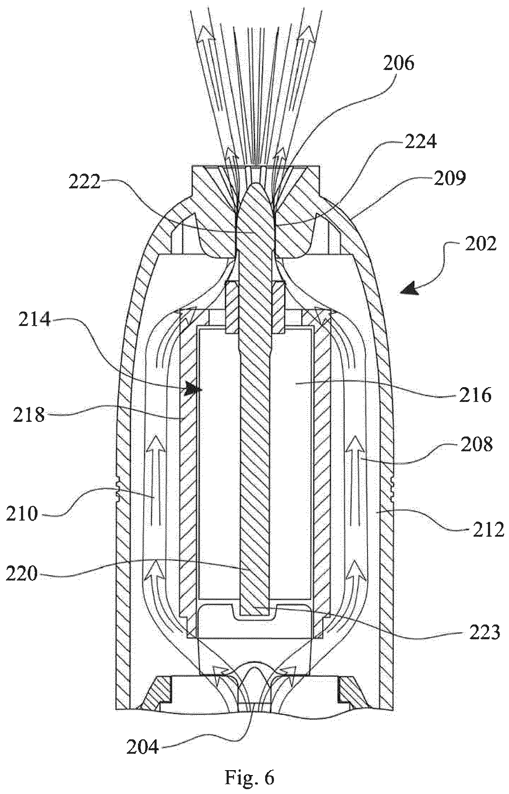

As a brief aside, it will be appreciated that the mass median aerodynamic diameter is a statistical measurement of the size of the particles/droplets in an aerosol. That is, the mass median aerodynamic diameter quantifies the size of the droplets that together form the aerosol. The mass median aerodynamic diameter may be defined as the diameter at which 50% of the particles/droplets by mass in the aerosol are larger than the mass median aerodynamic diameter and 50% of the particles/droplets by mass in the aerosol are smaller than the mass median aerodynamic diameter. The "size of the aerosol", as may be used herein, refers to the size of the particles/droplets that are comprised in the particular aerosol. Referring to Fig. 6, there is shown a flavour pod portion 202 of a consumable, the consumable providing an aerosol delivery device in accordance with the invention. The consumable further comprises a cartomiser portion (not shown in Fig. 6) having all of the features of the cartomiser portion 104 described above with respect to Fig. 5.

The flavour pod portion 202 comprises an upstream (i.e. upstream with respect to flow of air in use) inlet 204 and a downstream (i.e. downstream with respect to flow of air in use) outlet 206. Between and fluidly connecting the inlet 204 and the outlet 206 the flavour pod portion 204 comprises an airflow passage 208. The airflow passage 208 comprises a first airflow branch 210 and a second airflow branch 212, each of the first airflow branch 210 and the second airflow branch 212 fluidly connecting the inlet 204 and the outlet 206. In other examples the airflow passage 208 may have an annular shape. The outlet 206 is located at the mouthpiece 209 of the consumable 103, and is also referred to as a mouthpiece aperture 206.

The flavour pod portion 202 comprises a storage 214, which stores a first aerosol precursor. The storage 214 comprises a reservoir 216 located within a chamber 218. The reservoir 216 is formed of a first porous material.

The flavour pod portion 202 comprises a member 220, which comprises an aerosol generator portion 222 and a supporting portion 223. The aerosol generator portion 222 is located at a downstream end (an upper end in Fig. 6) of the member 220, while the supporting portion 223 makes up the rest of the member 220. The supporting portion 223 is elongate and substantially cylindrical. The aerosol generator portion 222 is bulb-shaped, and comprises a portion which is wider than the supporting portion 223. The aerosol generator portion 222 tapers to a tip at a downstream end of the aerosol generator portion 222.

The member 220 extends into and through the storage 214. The member 220 is in contact with the reservoir 216. More specifically, the supporting portion 223 extends into and through the storage 204 and is in contact with the reservoir 216. The member 220 is located in a substantially central position within the reservoir 216 and is substantially parallel to a central axis of the consumable. The member 220 is formed of a second porous material.

The first and second airflow branches 210, 212 are located on opposite sides of the member 220. Additionally, the first and second airflow branches 210, 212 are located on opposite sides of the reservoir 216. The first and second airflow branches 210, 212 branch in a radial outward direction (with respect to the central axis of the consumable 200) downstream of the inlet 204 to reach the opposite sides of the reservoir 216.

The aerosol generator portion 222 is located in the airflow passage 208 downstream of the first and second airflow branches 210, 212. The first and second airflow branches 210, 212 turn in a radially inward direction to merge at the member 220, at a point upstream of the aerosol generator portion 222.

The aerosol generator portion 222 is located in a narrowing section 224 of the airflow passage 208. The narrowing section 224 is downstream of the point at which the first and second airflow branches 210 212 merge, but upstream of the mouthpiece aperture 207. The mouthpiece aperture 207 flares outwardly in the downstream direction, such that a width of the mouthpiece aperture 207 increases in the downstream direction.

In use, when a user draws on the mouthpiece 209, airflow is generated through the airflow passage 208. Air (comprising the second aerosol from the cartomiser portion as explained above with respect to Fig. 5) flows through the inlet 204 before the air flow splits to flow through the first and second airflow branches 210, 212. Further downstream, the first and second airflow branches 210, 212 provide inward airflow towards the member 220 and the aerosol generator portion 222.

As air flows past the aerosol generator portion in the narrowing section 224, the velocity of the air increases, resulting in a drop in air pressure. This means that the air picks up the first aerosol precursor from the aerosol generator portion 222 to form the first aerosol. The first aerosol has the particle size and other properties described above with respect to Fig. 5.

As the first aerosol precursor is picked up by the air, the member 220 transfers further first aerosol precursor from the storage 214 to the aerosol generator portion 222. More specifically, the member 220 wicks the first aerosol precursor from the storage 214 to the aerosol generator portion 223.

In other examples, the storage 214 comprises a tank containing the first aerosol precursor as free liquid, rather than the reservoir 216 and the chamber 218. In such examples, the member 220 still extends into the tank to transfer first aerosol precursor from the tank to the aerosol generator portion 223.

Further arrangements of the present invention will now be described, which arrangements incorporate one or more features of the aspects of the present invention. In the subsequent arrangements, the smoking substitute device 100 includes a consumable 103 in the form of a cartomiser, but does not include a flavour pod. However the smoking substitute device 100 of the subsequent arrangements may be modified to incorporate a flavour pod in a way similar to the arrangement of Figures 5 and 6.

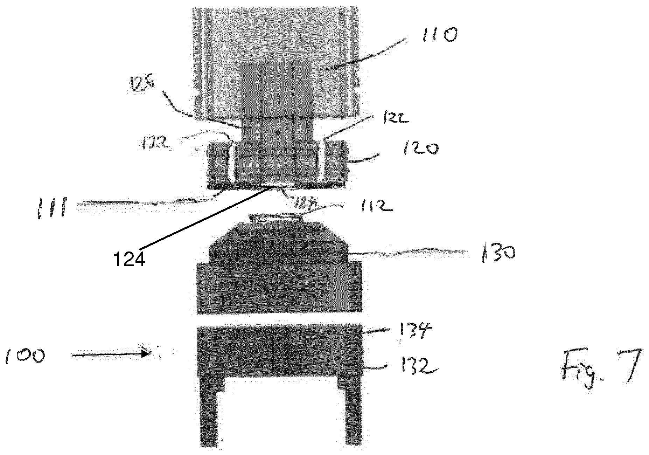

As mentioned above, the wick 111 is supported by a wick support element 120. Figure 7 illustrates an arrangement of a smoking substitute system in which these components are illustrated in more detail, and in an exploded view. The wick support element 120 is mounted at an end of the second storage reservoir 111 and has bores 122 therethrough to allow aerosol precursor in the second storage reservoir 110 to pass to the wick 110. These bores may be sized so that aerosol precursor may flow therethrough in a non-capillary manner. Although, two bores 122 are visible in Figure 7, there may be more arranged around the wick support element 120.

In the arrangement of Figure 7, the wick support element 120 is made of a resilient material, such as rubber, and thus may deform when force is applied thereto. In particular, when the consumable 103 is mounted on the main body 100, the wick 111 is brought into contact with the heater 112, and is held thereto by the resilience of the wick support element 120. The wick support element 120 may be sized so that it deforms slightly when the wick 111 is in contact with the heater 112, so as to provide a biasing force to urge the wick 111 into firm contact with the heater 112.

The wick 111 has an opening 124 at its centre, which is aligned with a passageway 126 through the wick support element 122. The passageway 126 communicates with the air-flow passage 108 shown in Fig. 5 so that air, together with vapour or a mixture of vapour and aerosol, will pass to the user. The surface of the wick 111 closest to the heater 112 acts as an activation surface for the aerosol precursor and, as the wick 111 is heated by the heater 112, aerosol precursor is released from the activation surface in the form of vapour or a mixture of vapour and aerosol, it can then pass through the opening 124 and the passageway 126 into the air-flow passage 108.

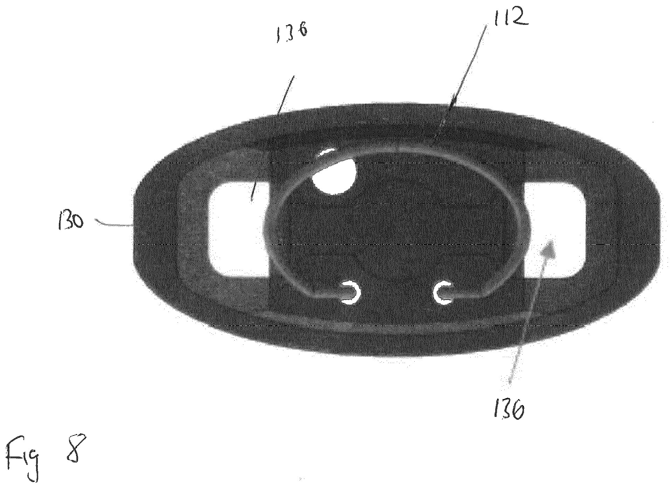

As illustrated in Figure 7, the heater 112 is mounted on a heater support element 130, which may act as an end wall of a battery housing and which may itself be supported by a support wall 132. The casing of the main body 100 (not shown in Figure 7) will enclose the support wall 132 and parts of the heater support element 130. In order for air to flow from the activation surface of the wick 111 through the opening 124 and into the passage 126, air must first reach the activation surface of the wick 111. The support wall 132 may thus have a bore 134 therethrough, which communicates with passages 136 (not shown in Figure 7) through the heater support element 130. Figure 8 illustrates these passages 136 and shows that they open immediately adjacent the heater 112 and hence adjacent the activation surface of the wick 111. The casing of the main body 100 may be provided with an inlet at a suitable location, to allow air to reach the bore 134, and hence to flow to the passages 136 in the heater support element 130. Hence, when the user draws on the mouthpiece 109 of the consumable 103, air is drawn into the casing of the main body 100 through the bore 134 and the passages 136 to reach the activation surface of the wick 111 adjacent the heater 112. That air then passes, together with vapour or mixture of aerosol and vapour generated by heating of the aerosol precursor by the heater 112, through the opening 124 in the wick 111 to the passage 126, and hence to the air-flow passage 108, and then to user, as has previously been described.

Note that in the arrangement of Figures 7 and 8, the heater 112 will need to be connected to a power source, such as a battery, and there may then need to be additional bores (not shown in Figures 7 and 8) through the heater support element 130 and the support wall 132 to allow electrical leads to pass therethrough.

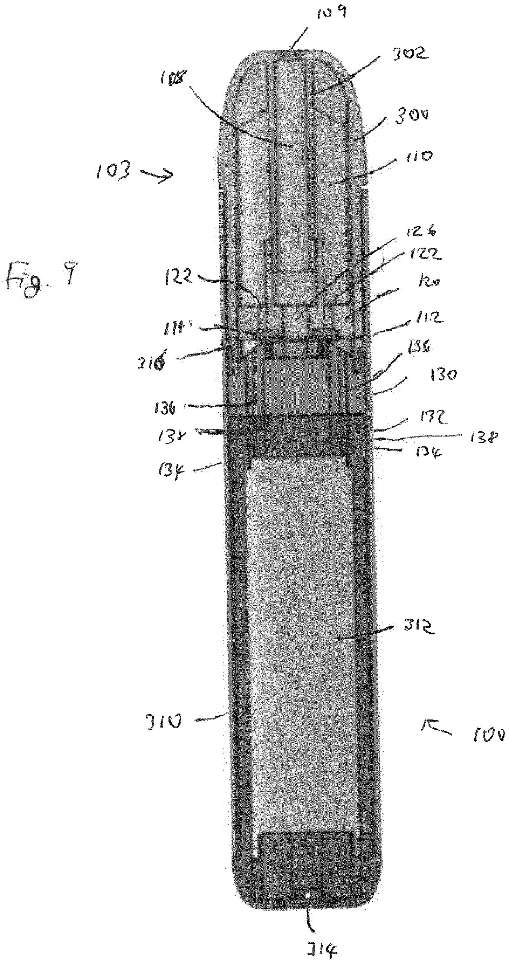

Figure 9 illustrates another arrangement of a smoking substitute system, in which the consumable has a single reservoir for aerosol precursor which corresponds to the second storage reservoir 110 in the embodiment of Figure 5. In this arrangement, the consumable does not have a flavour pod portion. For simplicity, parts corresponding to those of Figures 5 to 8 are indicated by the same reference numerals. Note that in Figure 9, the support wall 132 has multiple bores 134 therethrough, aligned with the passages 136 in the heater support element 130.

Figure 9 also shows the casings of the device. In particular, there is a casing 300 (the "first" casing), being a casing of the consumable 103. That casing contains the reservoir 110 for aerosol precursor, and also supports the wick support element 120 and the wick 111. A tube 302 within that first casing 300 forms a bounding wall of the air-flow passage 108, and the mouthpiece 109 is formed at an end of the first casing 300. The main device 100 also has a casing 310 (the "second" casing) on which are mounted the support wall 132 and the heater support element 130. There is a space 312 within the second casing 310 for a battery and other electronic components used to power the heater 112, and the second casing 310 may also have an inlet 314 to allow air to enter the space 312 and hence pass to the bores 134 and the passages 136 to enable it to reach the activation surface of the wick 110.

Figure 9 also shows electrical leads 138 which extend through the support wall 132 and the heater support element 130 to enable the heater 112 to be connected to a battery in space 312. Small bores may be formed in the heater support element 130 and the support wall 132 through which the leads 138 may pass. The first and second casings 300, 310 are separable and held together by a "click" engagement 316. When the two casings 300,310. are interconnected, as shown in Fig.9, the wick 111 is forced into contact with the heater 112 by the resilience of the wick support element 120, so that good heating of the activation surface of the wick 111 will occur when the heater 112 is active. The separability of the two casing 300, 310 allows the consumable 103 to be removed from the main body 100, and replaced, e.g. when the aerosol precursor in the reservoir 110 is exhausted.

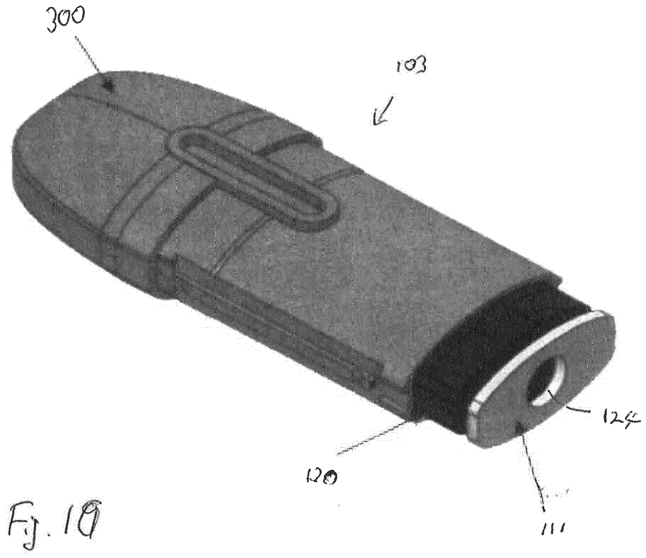

Figure 10 shows a perspective view of the consumable 103 in Figure 9, with the part of the first casing 300 removed so that the wick 111 and the wick support element 120 are clearly visible. It can be seen from Figure 10 that the wick 111 is flat and so has a planar activation surface (the exposed surface of the wick 111 in Figure 10). Figure 10 also shows clearly the opening 124 in the wick 111, which allows communication with the passageway 126 through the wick support element 120. The wick support element 120 in this embodiment, and in some other embodiments, is preferably made of rubber material. In a similar way, the wick 111 is preferably made of silica material, which material is suitably porous to allow the aerosol precursor to pass therethrough. Alternatively, the wick may be of fibrous material, woven material or porous ceramic material.

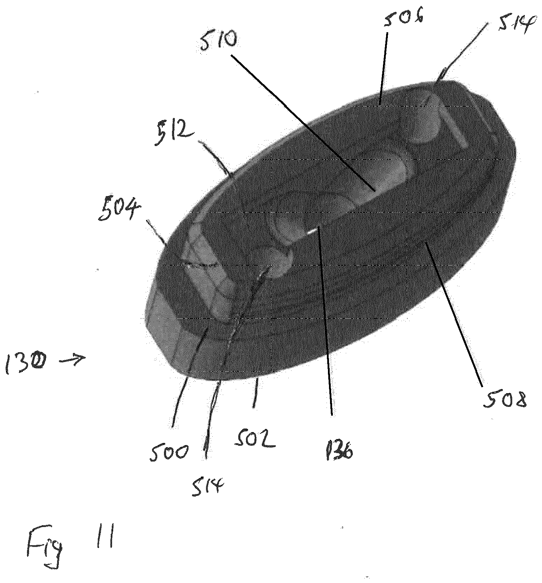

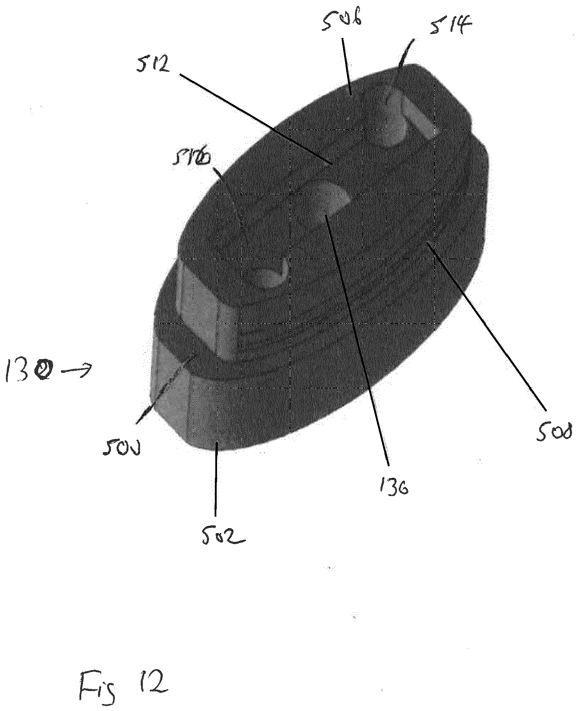

Figures 11 and 12 illustrate two alternative configurations of a heater support element 130 which may be used in the present invention. They differ in the shape of the mouth of the passage 136 through the heater support element 130 which allows air to pass through the heater support element from e.g. the interior of the casing of the main body 100 to the vicinity of the heater 112 and the activation surface of the wick 111. Note that, in Figures 11 and 12, the heater itself is not shown and there is a single passage 134 through the heater support element 132. In each of the alternative configurations, the heater support element 130 is preferably made of resilient material, which must also be suitable to resist the heat generated by the heater 112.

In Figure 11, the heater support element 130 comprises a body part 500 which has a peripheral seal surface 502 which seals to the casing 310 (not shown in Figure 11). The seal between the seal surface 502 and the casing 310 needs to be sufficiently strong to prevent, or at least significantly resist, movement of the heater support element 130 in the casing 310, particularly when the consumable 103 is removed from the main body 100.

A projecting part 504 projects from the body part 500, terminating in a flat heater support face 506. The periphery of the projecting part 504 seals to the casing 300 of the consumable 103, and for this purpose may have ribs 508 on its side surface. However, unlike the sealing of the seal surface 502 to the casing 310 of the main body 100, the sealing of the projecting part 504 to the casing 300 of the consumable 103 needs to allow the consumable 103 to be removed to allow another consumable 103 to be mounted thereon without too much resistance. Nevertheless, the sealing must be sufficiently good to limit leakage of any aerosol precursor which has passed through the wick 111 but has not been vaporised by the heater 112. As in the arrangement of Figure 9, the passage 136 passes through the heater support element 130 to enable air to pass towards the heater 112 and the wick 111. In the heater support element 130 shown in Figure 11, the passage 136 terminates in a splayed or funnelled mouth 510, which opens into a slot 512 in the heater support surface 506, so that air which has passed through the bore 136 can expand in the funnelled mouth 510 before reaching the heater 112.

Figure 11 also shows bores 514 through which pass leads from the heater 112, which leads will provide electrical connection to the battery.

The heater support element 130 shown in Figure 11 is resilient and is preferably made of silicone material, with provision to resist high temperatures which may be generated by the heater 112. For example, the material known as Polygraft HT-3120 silicone, which is a two-part mix, may be a suitable material from which the heater support element 132 may be made. The configuration shown in Figure 11 will normally be made by moulding the silicone material in a suitable mould.

Figure 12 illustrates an alternative heater support element 130. It is generally similar to the heater support element 130 shown in Figure 11 and the same reference numerals indicate corresponding parts. It may be made of the same materials as the heater support element 130 of Figure 11. The heater support element 130 of Figure 12 differs from that of Figure 11 in that the passage 136 opens directly into the channel 512 in the heater support surface 506. There is thus a flat face 516 at the bottom of the channel 516, rather than the funnel mouth 510 shown in Figure 11.

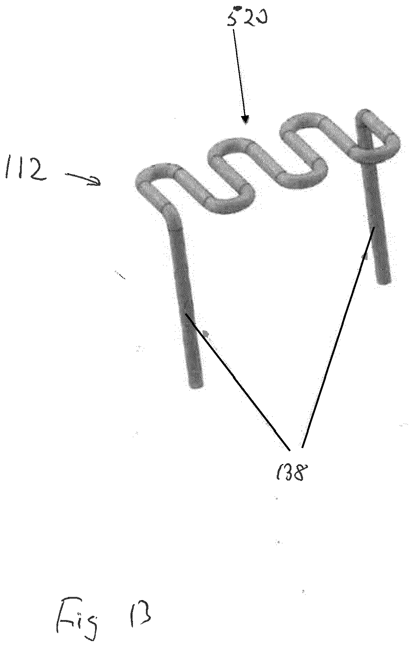

Figure 13 shows a heater that may be used with the heater support element 130 shown in Figure 11 or Figure 12. The heater comprises a heater filament 520 which is generally flat and rests on the heater support face 506 of the heater support element 130. For this reason, the filament 520 is not straight but meanders in its plane. Figure 13 also shows the leads 138 which extend through the bores 514 of the heater support 130 shown in Figure 11 or Figure 12, to enable the heater 112 to be connected to a battery.

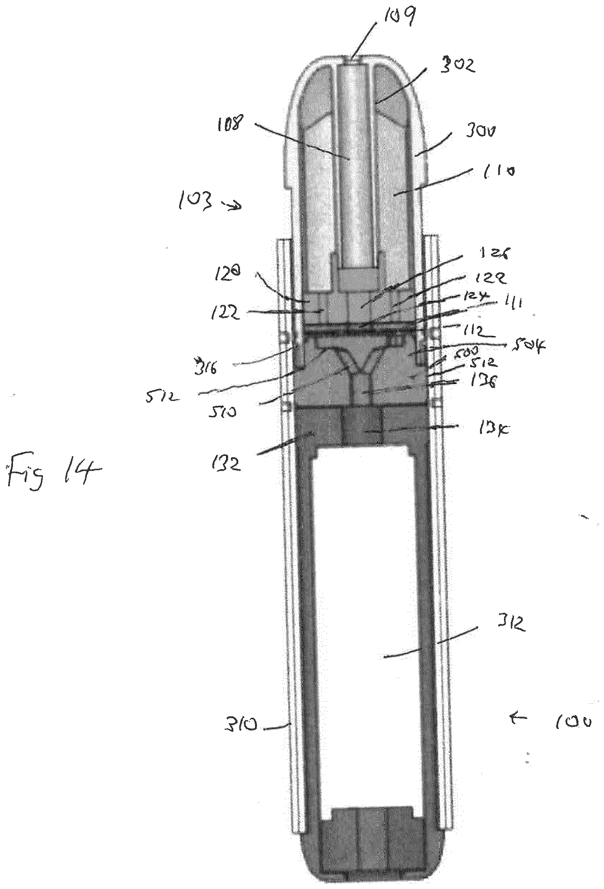

Figure 14 illustrates an arrangement of a smoking substitute system which incorporates the heater support element 132 of Figure 11, and also the heater 112 of Figure 13. The arrangement of Figure 14 is generally similar to that of Figure 9, and corresponding parts are indicated by the same reference numerals. As mentioned previously, when the heater support element 132 of Figure 11 is used, there is only a single bore 136 therein for air, hence there is only a single bore 134 in the support 132 in the main body 100. The bore 136 extends to the funnelled mouth 510 which opens into the slot 512 directly below the heater 112. Note that the leads 138 of the heater 112 are not visible in Figure 14.

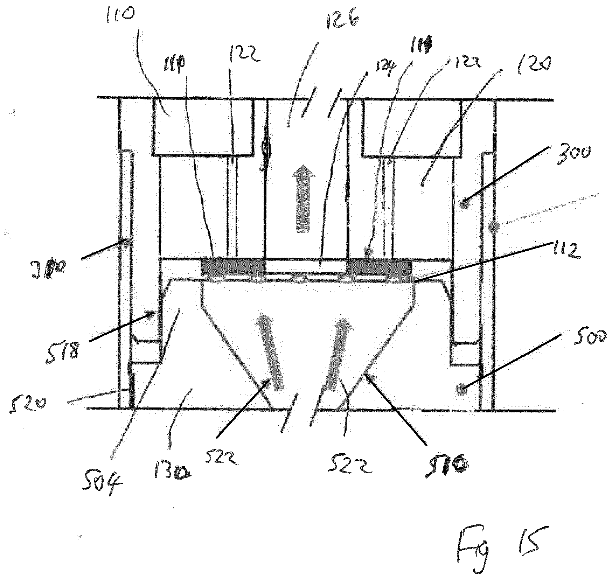

Figure 14 illustrates how the seal surface 502 of the main body 500 seals to the second casing 310, and the projecting part 504 seals to the first casing 300. This sealing is illustrated in more detail in the enlarged view of Figure 15. In particular, the first casing 300 of the consumable 103 extends sufficiently far within the second casing 310 of the main body 100 so as to contact the projecting part 504 of the heater support element 130 at a sealing interface 518. Similarly, the main body 500 of the heater support element 130 seals at a sealing interface 520 with the casing 310 of the main body 100. As mentioned previously, the degrees of sealing at these two sealing interfaces 518 and 520 are preferably different, since the heater support element 130 does not normally release from the second casing 310, but must release from the first casing 300 when the consumable 103 is removed.

Figure 15 also shows how the funnelled mouth 510 of the passage 136 opens within the heater support element 130 towards the heater 112 and the wick 111. This causes the airflow from the passage 136 to expand, as illustrated by the arrows 522, so that there is a good air flow where the heater 112 meets the wick 111, to entrain vapour therein prior to flow to the passage 126 in the wick support element 120.

With the arrangement shown in Figure 15, as in the other arrangements, the sealing between the first casing 300 and the heater support element 130 at the sealing interface 518 prevents any leakage of aerosol precursor which has come from the wick 111 and has not been vaporised by the heater 112. Hence, when the consumable 103 is fitted in place on the main body 100, the only escape route for the aerosol precursor is via the air flow passage 108 and the mouthpiece 109. This helps to ensure efficient consumption of the aerosol precursor.

The arrangement of Figure 14 also differs from the arrangement of Figure 9 (and also that of Figure 15), in that the wick 111 extends across the whole of the end face of the wick support element 120, as in the arrangement of Figure 10. As before, the wick 111 has an opening 124 therein to allow air to pass through the wick 111 and into the passage 126, and hence through the air-flow passage 108 so that it can reach the outlet 109 and thus pass to the user.

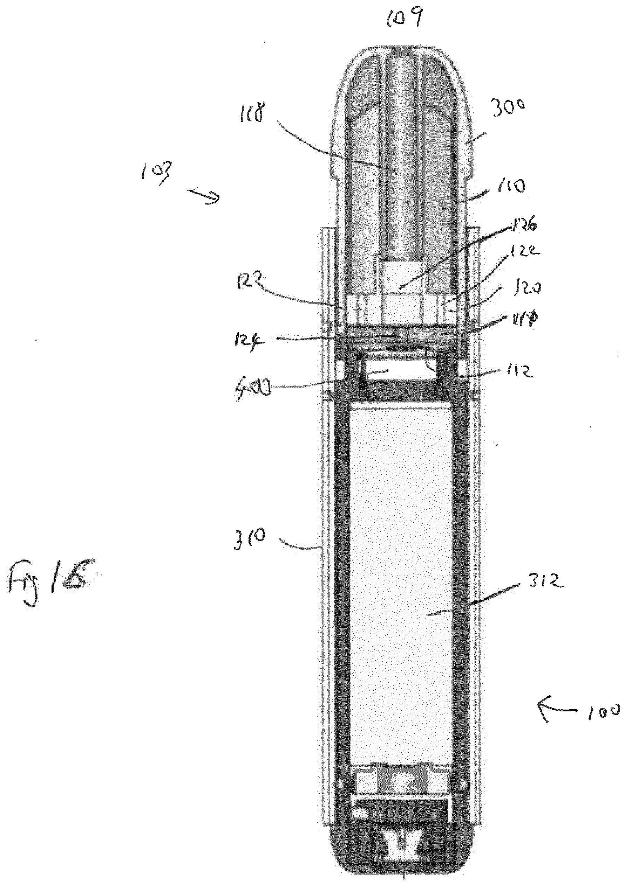

Figure 16 shows another arrangement of a smoking substitute system, which is generally similar to that of the embodiment of Figs. 9 and 10 and corresponding parts are indicated by the same reference numerals. In the embodiment of Fig.16, however, there is no heater support element 130, and instead the heater 112 is a coil or other filament held within the second casing 310, which has a space 400 adjacent thereto. The space 400 communicates with inlets (not shown in Figure 16) which allow air to enter the casing 310 and pass to the activation surface of the wick 111. Again, the wick 111 is forced into contact with the heater 112 by the resilience of the wick support element 120. In this arrangement, the flow of air to the activation surface is not restricted by the size of the passage or passages through the heater support element 130. In this arrangement the heater 112 needs to be sufficiently stiff that it is not deformed when the wick 111 is urged into contact therewith by the resilient wick support element 120.

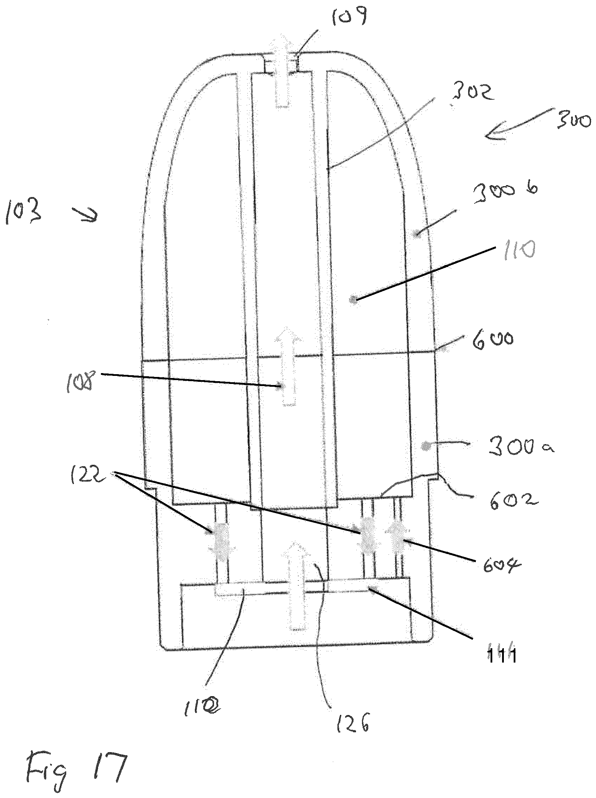

In the arrangements of the smoking substitute system described above, the wick support element 120 is a separate element from the first casing 300 of the consumable 103. Figure 17 illustrates an alternative arrangement, in which the wick support element is integral with part of the first casing 300.

In the arrangement of Figure 17, parts which correspond to arrangements described previously are indicated by the same reference numerals. Note that, in Figure 17, the main body 100 is not shown. It may be the same as in the other arrangements of a smoking substitute system described previously.

In the arrangement of Figure 17, the first casing 300 has a lower part 300a and an upper part 300b. The mouthpiece 109 is in the upper part 300b, and the tube 302 is also integral with that upper part 300b. The lower part 300a has an upper rim which meets a lower rim of the upper part 300b at a sealing surface 600, and has an internal flange 602 adjacent its lower end. The internal flange 602 corresponds to the wick support element 120 of the arrangements previously described. The internal flange 602 has a central bore forming passage 126, which passage is aligned with the passage 108 within the tube 302. The end of the tube 302 furthest from the mouth piece 109 engages the flange 602 and is sealed thereto.

The interiors of the upper and lower parts 300b and 300a of the casing 300 are hollow, and form the reservoir 110. There are bores 122 in the flange 602 to allow the reservoir 110 to communicate with the wick 111, in the same way as the bores 122 in the earlier arrangements described previously. Thus, aerosol precursor in the reservoir 110 may pass through the bores 122 to saturate the wick 111, and then be heated by the heater 112 (not visible in Figure 17). The arrangement of Figure 17 prevents any leakage of aerosol precursor between the wick support element 120 and the casing 300. Whilst there could be leakage between the upper and lower parts 300b, 300a of the casing 300, this can be prevented by suitable configuration of the sealing interface 600. However, if the sealing of the reservoir 110 is too good, air may not be able to enter it to replace aerosol precursor which has been consumed.

Therefore, Figure 17 shows that there may be at least one additional bore 604 in the flange 602, to allow passage of air to the reservoir 110 from outside the first casing. The or each additional bore 604 needs to be sufficiently small that it will not allow a significant amount of aerosol precursor to pass therethrough. For example, the or each additional bore 604 may be e.g. 0.2 to 0.5mm in diameter, more preferably 0.32 to 0.5mm, even more preferably 0.32 to 0.4mm. If the flange has a thickness of e.g. 0.5 to 5mm, preferably 1 to 5mm, aerosol precursor should not be able to escape reservoir 110 through the or each additional bore 604. In general, the thicker the flange 602, the greater the possible diameter of the or each additional bore 604 may be, without it allowing aerosol precursor to flow therethrough. A thin flange 602 (which thinness may be desirable for manufacture) will thus need the diameter of the or each additional bore to be small.

The upper and lower parts 300a, 300b of the casing 300 may be separable to allow for refiling of the reservoir 110 once the aerosol precursor wherein has been consumed. In such an arrangement, the sealing at the sealing surface 640 needs to be sufficiently good to prevent leakage of aerosol precursor therethrough when the smoking substitute system is in use. Alternatively, the seal at the sealing surface 600 may be a permanent one, with the upper and lower parts 300a and 300b if the casing bonded together. In such an arrangement, the reservoir 110 may not be refillable, and the consumable 101 would need to be replaced once the aerosol precursor in the reservoir 110 had been consumed.

In the arrangements described previously, the bores 122 in the wick support element 120 (or in the flange 602 in the case of Figure 17) were described as being sized so that aerosol precursor may flow therethrough in a non-capillary manner. In an alternative, applicable to all the arrangements described previously, the bores 122 may be capillary ducts (hereinafter referred to as capillary bores) which allow aerosol precursor to flow therethrough in a capillary manner. The capillary bores allow the flow of aerosol precursor to the wick 111, in a controlled manner, so that there is less chance of there being excess aerosol precursor at the wick 111. In general, the capillary bores may have a diameter range of 0.3mm to 2mm, as a diameter of less than 0.3mm will generally not allow sufficient aerosol precursor to pass to the wick 111. Preferably, the diameter is at least 0.5mm, preferably 0.8 to 1.5mm, and more preferably 1 mm or 1.3mm. In practice, the diameter of the capillary bores may be affected by the thickness of the wick support element 120, which can have a thickness of e.g. 0.5mm to 5mm, more preferably 1 to 5mm, such as 4mm, 3mm, 2mm and 1mm. In general, the width of the capillary bores will need to be greater with greater thickness of the wick support element 120.

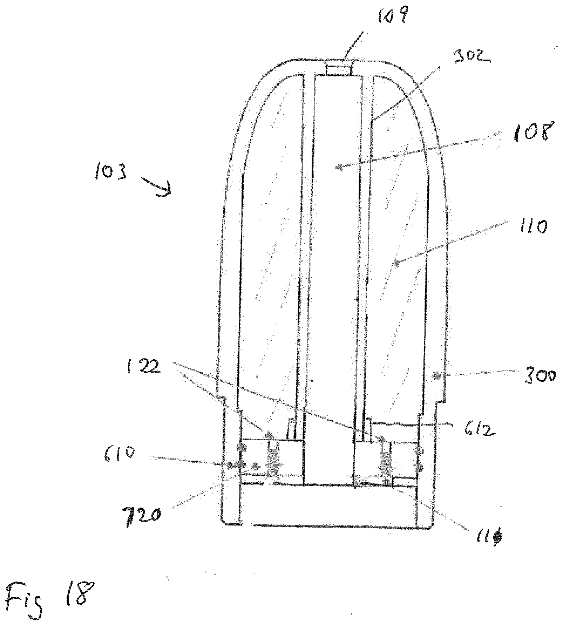

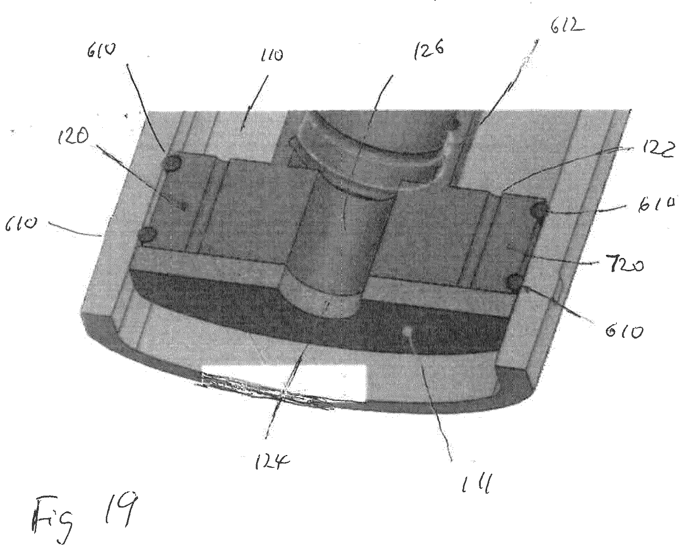

In the arrangements of Figures 6 to 16, the wick support element 120 is made of resilient material such as rubber. In the arrangement of Figure 17 on the other hand, the support for the wick 111 is rigid, because it was formed by the internal flange 602 which was integral with, and therefore made of the same material as, the casing 300. Figures 18 and 19 then illustrate another arrangement in which the wick is supported by a rigid element. Unlike the arrangement of Figure 17, however, in the arrangement of Figures 18 and 19, that rigid element is a separate wick support element 720. In Figures 18 and 19, parts which correspond to parts of earlier arrangements are indicated by the same reference numerals. Moreover, as in Figure 17, only the consumable 103 is illustrated. The main part 100 may be the same as in earlier arrangements.

In particular, in the arrangements of Figures 18 and 19, the rigid wick support element 720 is formed at an end of the reservoir 110, within the first casing 300. Bores 122 through the wick support element 720 allow aerosol precursor from the reservoir 110 to pass to wick 111. Whilst the bores 122 may be non-capillary bores, they are preferably capillary bores. The diameter of the capillary bores may be as previously described, as may the thickness of the wick support element 720. Although not illustrated in Figures 18 and 19, there may need to be an additional bore or bores in the wick support element 720 to allow passage of air to the reservoir 110, corresponding to the at least one additional bore 604 in Figure 17.

In order to prevent escape of liquid from the reservoir, the wick support element 720 is preferably sealed to the first casing 300 by seals 610. For example, the seals 610 may be O-ring seals extending around the wick support element 120. The seals can be seen clearly in Figure 19, as can the opening 124 in the wick 111, which leads to the passage 126 through the wick support element 720 to the air-flow passage 108. The wick support element 720 also needs to be sealed to the tube 302, to prevent escape of aerosol precursor from the reservoir 110. To achieve this, the wick support element 720 may have an upstanding ring 612, which then seals (e.g. by O-rings and/or an interference fit) to the tube 302. Grooves for those O-rings are illustrated in Figure 19. Another possibility is for the tube 302 to be integral with the wick support element 720, with the end of the tube 302 being sealed to the casing 300 adjacent the mouthpiece 109.

The rigidity of the wick support element 720 and the tube 302 means that the positioning of the wick support element 720 on the tube 302 and the positioning of the tube 302 relative to the casing 300 may be determined to good precision. This ensures that the wick 111 is accurately positioned relative to the casing 300, and hence accurately positioned relative to the casing 310 and the heater 112.

In the arrangement of Figures 18 and 19, the wick support element 720 may be made of the same material as the casing 300 (and the casing 310) such as being made from moulded polypropylene plastics material. Other suitable materials to form the wick support element 720 include ABS and PEAK materials. The seals 610 may be O-rings of e.g. rubber material or silicone seals co-moulded with the wick support element 720, but preferably are nitrile or thermoplastic polymer O-ring seals. The moulding of the wick support element 720 and the first and second casings 300, 310 simplifies manufacture.

Because the wick support element 720 is rigid in the arrangement of Figures 18 and 19, it may be thinner than the resilient wick support elements 120 described with reference to e.g. Figures 5 to 16. Thus, it may then be possible to have a wick support element 720 with a thickness of e.g. 0.5 to 2mm, preferably 1mm, allowing the bores 122 to have a small diameter, and still provide a capillary effect. The same is true in the arrangement of Figure 17. Thus, at least in the arrangements of Figures 17 to 19, the bores 122 may have a diameter of 0.3mm to 2mm, most preferably 0.5mm. If one or more additional bores are provided, corresponding to the additional bores 604 in the arrangement of Figure 18, to allow air to enter the reservoir volume to replace aerosol precursor which has passed to the wick 111, those additional bores will have small diameters, due to the reduced thickness of the wick support element 720, so e.g. less than 0.3mm. The diameter of the additional bores will always be less than the diameter of the capillary bores. It should be noted that, even in the arrangements of Figures 5 to 16, it may be possible to have small diameter capillary bores, if the wick support element 120 s thin enough.

In the arrangements of Figures 17 to 19, the position of the wick 111 is precisely determined, relative to the casing 300, either because the wick support element is part of the casing itself, as in the arrangement of Figure 17, or because the position of the wick support element 720 is determined by a component of the casing such as the tube 302, as in the arrangement of Figures 18 and 19. This precise positioning of the wick 111 in the casing 300 means that manufacture will be consistent and hence replacement of one consumable with another will not alter the relationship between the wick 111 and the heater 112, and so will not affect the efficiency of the smoking substitute device.

The use of capillary bores 122 in the wick support element 720 in the arrangements of Figures 17 to 19 mean that it is possible to optimise the flow of aerosol precursor to the wick 111 to minimise leakage. The length and diameter of the capillary bores 122 may be chosen to control the flow of a specific aerosol precursor formulation to the wick 111, based on the viscosity and liquid characteristics of that aerosol precursor. When aerosol precursor is vaporised from the wick 111 by the heater 112, there will be an available volume of air in the wick 111 allowing additional aerosol precursor to flow into the wick 111, so that the wick 111 is maintained in a saturated state when the device is in use. The rigid nature of the wick support element 720 improves the consistency of liquid flow to the wick 111, compared to a wick support element 120 of resilient material, so that efficient operation may be achieved.

The sealing configuration in the arrangement of Figures 18 and 19 makes use of O-rings, with the effect of minimising leakage in use and in transit, as a robust seal is created between the wick support element 720 and the casing 300, so that there is no leakage path therebetween. O-ring technology is well established, so it is straight forward to put in to practice in the smoking substitute device to reduce or eliminate variation between parts, improving repeatability of manufacture.

The use of a rigid wick support element 720 in the arrangements of Figures 17 to 19 means that the wick support element 720 is easy to manufacture with high precision, and the assembly of the consumable may easily be automated. This ensures efficient manufacture, thereby reducing costs.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. An aerosol delivery device comprising a heater, a fluid-transfer article and a first casing, the fluid-transfer article comprising a reservoir for holding an aerosol precursor, a wick arranged to receive aerosol precursor from said reservoir, and a wick support element; wherein said wick support element is arranged to support said wick such that said heater makes abutting unbonded contact with an activation surface of said wick so as to interact thermally with said activation surface; and wherein said first casing contains said reservoir therein and said wick support element is integral with said first casing, said wick support element having at least one bore therethrough for passage of said aerosol precursor from said reservoir to said wick.

2. An aerosol generation apparatus according to claim 1, wherein said activation surface of said wick is planar.

3. An aerosol delivery device according to claim 1 or claim 2, wherein said at least one bore is sized so as to define at least one non-capillary duct through said wick support element, whereby said aerosol precursor is able to flow in a non-capillary manner from said reservoir to said wick.

4. An aerosol delivery device according to claim 1 or claim 2, wherein said at least one bore is sized so as to define at least one capillary duct through said wick support element, whereby said aerosol precursor is able to flow in a capillary manner from said reservoir to said wick.

5. An aerosol delivery device according to claim 4, wherein the or each capillary duct has a diameter of at least 0.3mm.

6. An aerosol delivery device according to claim 5, wherein the or each capillary duct has a diameter of at least 0.5mm

7. An aerosol delivery device according to any one of claims 4 to 6, wherein the or each capillary duct has a diameter not greater than 2mm.

8. An aerosol delivery device according to any one of claims 4 to 6, wherein the or each capillary duct has a diameter of 0.8 to 1.5mm.

9. An aerosol delivery device according to any one of the preceding claims, wherein said wick support element has a thickness of at least 0.5mm between said reservoir and said wick.

10. An aerosol delivery device according to claim 9, wherein said wick support element has a thickness of at least 1mm between said reservoir and said wick.

11. An aerosol delivery device according to any one of the preceding claims, wherein said wick support element has a thickness not greater than 5mm between said reservoir and said wick

12. An aerosol delivery device according to any one of the preceding claims, wherein said wick support element forms an end wall of said reservoir.

13. An aerosol delivery device according to any one of the preceding claims, wherein said wick is of silica material.

14. An aerosol delivery device according to any one of the preceding claims, wherein said first casing comprises first and second casing parts, each of said first and second casing parts being hollow and containing said reservoir therein, said wick support element being integral with said second casing part.

15. An aerosol delivery system according to claim 14, wherein said first and second casing parts are separable.

16. An aerosol delivery device according to any one of the preceding claims further including a second casing supporting said heater, wherein said first and second casings are separably interconnected.

17. An aerosol delivery device according to claim 16, wherein said first casing has an outlet, and there is a first air-flow pathway from said activation surface to said outlet.

18. An aerosol delivery device according to claim 17, wherein said wick and said wick support element have aligned openings therethrough, said aligned openings forming a part of said first air-flow pathway.

19. An aerosol delivery device according to any one of the claims 16 to 18, wherein said second casing has an inlet, and there is a second air-flow pathway from said inlet to said activation surface.
